# FASCICULE DE BREVET EUROPEEN

(11) **EP 1 014 948 B1**
(45) Date de publication et mention de la délivrance du brevet: **27.06.2007**
(21) Numéro de dépôt: 98943975.7
(22) Date de dépôt: 14.09.1998
(51) Int. Cl.: A61K 9/19, A61K 47/18, A61K 47/26

(54) **COMPOSITION PHARMACEUTIQUE PLACEBO LYOPHILISABLE DESTINEE A IMITER UN MEDICAMENT, NOTAMMENT A BASE DE PROTEINES OU DE POLYPEPTIDES**
LYOPHILISIERBARE PHARMAZEUTISCHE PLAZEBOZUSAMMENSETZUNG , INSBESONDERE AUF BASIS VON PROTEINEN ODER POLYPEPTIDEN, ZUR NACHAHMUNG EINES ARZNEIMITTELS
FREEZE-DRIED PLACEBO PHARMACEUTICAL COMPOSITION DESIGNED TO IMITATE A MEDICINE, IN PARTICULAR BASED ON PROTEINS OR POLYPEPTIDES

(30) Priorité: 18.09.1997 FR 9711626
(43) Date de publication de la demande: 05.07.2000
(73) Titulaire: Laboratoire Français du Fractionnement et des Biotechnologies, 91940 Les Ulis (FR)
(72) Inventeur: BARDAT, Annie, F-91470 Limours (FR); SCHMITTHAUESLER, Roland, F-78180 Montigny le Bretonneux (FR)
(74) Mandataire: Warcoin, Jacques
(86) Numéro de dépôt international: PCT/FR1998/001956
(87) Numéro de publication internationale: WO 1999/013866

(56) Documents cités:
- EP-A- 0 084 705
- EP-A- 0 211 257
- EP-A- 0 682 944
- FR-A- 2 751 177
- DATABASE WPI Week 7625 Derwent Publications Ltd., London, GB; AN 76-46915x XP002065798 & JP 51 051511 A (TATSUHARA), 7 mai 1976

## Description

L'invention concerne le domaine pharmaceutique et médical et vise à proposer le simulacre d'un médicament sous sa forme galénique habituelle, mais ne contenant pas le principe actif.

L'invention s'applique tout particulièrement aux formes galéniques lyophilisées simulant des médicaments contenant des peptides ou des protéines obtenus par extraction, à partir de fluides biologiques animaux ou humains, par recombinaison génétique, par synthèse ou modifiés par réactions chimiques dirigées ou par remodelage génomique ou par transgénie.

Ces leurres peuvent alors être utilisés pour des études cliniques contrôlées en double insu.

La mise sur le marché de médicaments est conditionnée par les résultats obtenus par ceux-ci dans le cadre d'études cliniques contrôlées, où l'expérimentateur se propose de mesurer les effets thérapeutiques des principes actifs à l'étude, par rapport à une substance-témoin ne contenant aucun principe actif appelée placebo. Par cet artifice, on évite l'influence de facteurs incontrôlables, fiés à la subjectivité des observations ou à l'état du patient. Dans ces études, il faut proposer un placebo dont l'aspect, la consistance, les caractères observables se rapprochent le plus possible du médicament réel. La fabrication de tels placebos pose de grandes difficultés lorsque la substance à étudier se présente sous la forme galénique d'un lyophilisat et qu'elle contient des principes actifs de nature protéique ou polypeptidique.

Par example, EP 211 257 décrit une composition lyophilisée pouvant être exempte de médicament et comprenant des acides aminés cristallins. De même EP 682 944 décrit une composition lyophilisée avec du mannitol et de l'alanine.

En effet, les protéines ou polypeptides ont un caractère amphiphile et modifient la tension superficielle de l'eau, solvant habituel, ce qui conduit à la formation d'une couche de mousse lors de la reconstitution du médicament. De plus, la structure macromoléculaire des protéines ou peptides donne au lyophilisat une tenue qui se visualise sous la forme d'un "gâteau" de substance sèche, plus ou moins épais, enfermé dans le flacon. Le placebo ne doit contenir aucun principe actif : seuls les excipients habituels du médicament à l'étude sont autorisés, accompagnés le cas échéant, d'une ou plusieurs autres substances inertes.

Jusqu'à présent, les difficultés étaient résolues en ajoutant une macromolécule inerte, type albumine humaine ou gélatine injectable. Les risques potentiels évoqués récemment, lors de l'irruption de maladies neurodégénératives chez les bovins et dues à des agents transmissibles de nature encore inconnue, ont conduit à ne plus tolérer ces substances macromoléculaires inertes dans la fabrication de placebo afin de ne pas faire encourir un risque non évaluable au receveur du placebo.

Il n'y a donc pas, dans l'état actuel de la technique, de moyen de formuler un simulacre de médicament sans principe actif de nature protéique ou polypeptidique, ressemblant de façon indiscernable au produit contenant le principe actif.

Pour remédier à cette difficulté, on a trouvé que la consistance du "gâteau" de lyophilisat de produit réel pouvait être imitée en lyophilisant une solution de ou des excipients habituels du produit (sucres, sels minéraux, acides aminés sous forme amorphe à l'état solide) en présence d'un acide aminé ou d'un saccharide facilement cristallisable, ajoutés en quantités telles que la composition de placebo reconstituée après lyophilisation demeure compatible avec l'isotonie requise pour une injection parentérale, intramusculaire, sous-cutanée ou autre application (percutanée, oculaire).

L'invention concerne donc une composition pharmaceutique lyophilisée destinée à imiter un médicament, notamment à base de protéines ou de peptides, exempte du médicament, et pouvant servir de placebo, caractérisée en ce qu'elle comprend l'excipient habituel dudit médicament, un ou plusieurs sucres-alcools sous forme cristalline et/ou un plusieurs acides aminés sous forme cristalline.

Généralement, l'excipient habituel du principe protéique ou polypeptidique est un sucre notamment choisi dans le groupe constitué par les oligosaccharides, les disaccharides, les monosaccharides. Parmi les monosaccharides, on préfère le glucose ou le fructose seuls ou en mélange. Parmi les disaccharides, on préfère le saccharose, le maltose ou le lactose seuls ou en mélange.

L'excipient habituel peut aussi être un sel ou un mélange de sels alcalins ou alcalino-terreux (citrate, phosphate, glutamate, acétate de calcium ou sodium), un ou plusieurs acides aminés sous forme amorphe à l'état solide, l'ajout d'une quantité appropriée d'acides aminés cristallisables rendant le mélange cristallin dans le produit lyophilisé selon l'invention. Dans le cas des médicaments placebos simulant des médicaments contenant des peptides ou des polypeptides, l'excipient ne doit comprendre ni peptide ni polypeptide.

La tenue du produit desséché peut être améliorée par des excipients de masse moléculaire élevée : dextran, hydroxyéthylamidon, polyéthylène glycol, cyclodextrine qui sont facilement lyophilisables, mais qui nécessitent une addition de sels pour maintenir l'osmolarité dans des valeurs acceptables pour un usage parentéral (particulièrement lorsqu'il s'agit d'injecter un grand volume de l'ordre de 50 à 100ml).

De préférence, la composition présente une osmolarité comprise entre 250 et 650 milliosmoles après reconstitution sous forme de solution.

Un procédé de préparation consiste à mélanger les différents ingrédients en solution puis à lyophiliser la solution obtenue. Lors de la préparation de la solution, les sucres ou acides aminés sont «cristallisables», terme que l'on utilisera par la suite dans ce contexte. Le terme « cristallin » ou « sous forme cristalline » est réservé au produit fini lyophilisé.

Parmi les sucres-alcools cristallisables, on préfère le mannitol, le sorbitol ou un mélange de ceux-ci.

Parmi les acides aminés cristallisables, on préfère la glycine l'alanine, la valine, la leucine, la lysine, l'acide glutamique, l'acide aspartique, l'arginine, l'histidine. Ces sucres-alcools ou acides aminés cristallisent facilement lors de la congélation de la solution de départ permettant de conduire à la composition lyophilisée.

De plus, selon un mode de réalisation préféré de l'invention, la présence de tensioactif non ionique compatible avec l'usage parentéral, en faible quantité, donne au placebo reconstitué une tenue de mousse comparable à celle du produit réel.

Parmi les agents tensioactifs non ioniques, on cite le polysorbate, de l'octoxynol, des alcools polyoxyéthyléniques ou des esters polyoxyléthyléniques d'acides gras. On cite notamment le Tween® 80.

Enfin, pour simuler l'aspect colloïdal de la solution réelle de protéines, il a été trouvé que l'addition contrôlée d'une huile métabolisable, pharmaceutiquement acceptable, ou d'un composé hydrolipidique emulsionné en faibles quantités donnait un effet Tyndall comparable à celui du produit réel.

On appelle Effet Tyndall, la diffusion de la lumière blanche dans la solution de macromolécules due à l'interaction lumière/protéines donnant l'aspect "colloïdal" à ce type de solution, reflet de sa polydispersité. Parmi les huiles pharmaceutiquement acceptables, on cite l'huile d'olive, de maïs, de tournesol, de soja, de ricin, de squalène ou d'alphatocophérol. Ces huiles sont compatibles avec la voie parentérale.

Une émulsion typique de lipides injectables pour administration parentérale peut également être utilisée (Intralipid®).

Certaines protéines à forte concentration possèdent une couleur légèrement jaune à brun. Les placebos obtenus selon l'invention, peuvent simuler cette couleur par addition de colorants pharmaceutiquement acceptables ou par addition de mixtures particulières obtenues par la réaction de Maillard entre un sucre réducteur et un acide aminé. Selon le sucre et l'acide aminé choisis, les nuances vont du jaune clair au brun foncé.

Les compositions ci-après sont données à partir du procédé de préparation en solution (étape qui sera suivie de la lyophilisation). Les proportions de la composition séchée peuvent être facilement déduites à partir des solutions.

Selon un mode de réalisation préféré, la composition est caractérisée en ce qu'elle est susceptible d'être obtenue à partir d'une solution de départ comprenant en pourcentage poids :
- excipient habituel 0,1 à 50% (PN)
- acide aminé ou sucre cristallisable 0,1 à 20% (PN)
- tensioactif 10 à 200 ppm
- huile métabolisable ou composé hydrolipidique émulsionné et pigment (quantité suffisante pour avoir l'aspect du médicament)

Avantageusement, la solution comprend en pourcentage poids:
- excipient habituel, inférieur ou égal à 5% (P/V)
- acide aminé ou sucre cristallisable 1 à 5% (PN)
- tensioactif 50 à 100 ppm
- huile métabolisable ou composé hydrolipidique émulsionné et pigment (quantité suffisante pour avoir l'aspect du médicament)

La composition lyophilisée est particulièrement adaptée pour se présenter sous forme injectable, après reconstitution ou pour application percutanée ou oculaire après reconstitution.

Un autre aspect de l'invention est la possibilité de formuler un mélange saccharide/excipient/acides aminés ou sucres-alcools de telle façon que le placebo puisse être produit selon un cycle de lyophilisation court, qui ne nuit pas à l'aspect final du produit desséché.

Un procédé de préparation d'une telle composition lyophilisée consiste à ajouter, dans les proportions souhaitées, les différents ingrédients, en solution, puis à soumettre la solution à un cycle de congélation-lyophilisation. Les exemples suivants aideront à la compréhension détaillée de l'invention, tout en ne limitant pas son champ d'application à la seule description des applications réalisées.

### Exemple 1

On réalise une solution contenant 50 g/l de saccharose et 30 g/l de mannitol. On ajoute 35 mg/l de Tween® 80. Après répartition par 10 ml sur une hauteur de 2 cm par flacon, la solution est congelée et lyophilisée pendant 48 heures.

Le placebo desséché ressemble au produit thérapeutique et se reconstitue par addition d'eau pour préparation injectable avec formation de mousse. Le placebo reconstitué présente une osmolarité de 340 milliosmoles.

### Exemple 2

On réalise une solution contenant 10 g/l de saccharose et 25 g/l de glycine. On ajoute 25 mg/l de Tween® 80. Après répartition par 10 ml sur une hauteur de 2 cm par flacon, la solution est congelée et lyophilisée pendant 48 heures.

Le placebo desséché ressemble au produit thérapeutique et se reconstitue par addition d'eau pour préparation injectable avec formation de mousse. Le placebo reconstitué présente une osmolarité de 354 milliosmoles.

### Exemple 3

On réalise une solution contenant 50 g/l de saccharose et 50 g/l de mannitol. On ajoute 60 mg/l de Tween® 80 et 0,065 ml/l d'Intralipid® à 10%. Après répartition par 180 ml sur une hauteur de 4,5 cm, la solution est congelée et lyophilisée pendant 90 heures.

Le placebo desséché ressemble au produit thérapeutique et se reconstitue par addition d'eau pour préparation injectable avec formation de mousse. La solution présente un aspect colloïdal. Le placebo reconstitué présente une osmolarité de 465 milliosmoles.

### Exemple 4

On réalise une solution contenant 10 mM de trisodium citrate et 50 g/l de mannitol. On ajoute 35 mg/l de Tween® 80. Après répartition par 10 ml sur une hauteur de 1 cm, la solution est congelée et lyophilisée pendant 35 heures.

Le placebo desséché ressemble au produit thérapeutique et se reconstitue par addition d'eau pour préparation injectable avec formation de mousse. Le placebo reconstitué présente une osmolarité de 334 milliosmoles.

### Exemple 5

On réalise une solution contenant 44 g/l de saccharose, 35 g/l de mannitol, 1,75 g/l de chlorure de sodium et 0,05 g/l de Tween® 80. Après répartition par 180 ml sur une hauteur de 4,5 cm, la solution est congelée et lyophilisée en 100 heures.

Le placebo desséché ressemble au produit thérapeutique et se reconstitue par addition d'eau pour préparation injectable avec formation de mousse. Le placebo reconstitué présente une osmolarité de 407 milliosmoles.

### Exemple comparatif

10 ml d'une solution de saccharose à 10% sont lyophilisés sur une épaisseur de 2 cm dans les conditions suivantes :
T° congélation: - 50°C
T°dessiccation primaire : - 38°C dans le produit
T°dessiccation secondaire : + 40°C
Durée du cycle : 180 heures

A la fin d'un tel cycle, le seul composé se retrouve sous forme d'un "gàteau". Le placebo reconstitué présente une osmolarité de 314 milliosmoles. Cependant on notera que la duré du cycle est de 180 heures, ce qui constitue une durée rédhibitoire pour toute application industrielle.

## Revendications

1. Composition pharmaceutique lyophilisée destinée à imiter un médicament, à base de protéines ou de peptides, ladite composition étant exempte desdites protéines ou desdits peptides et étant destinée à servir de placebo, **caractérisée en ce qu'**elle comprend un excipient habituel choisi parmi les acides aminés qui se présentent à l'état solide sous forme amorphe, un ou plusieurs sucres-alcools sous forme cristalline et/ou un plusieurs acides aminés sous forme cristalline, et un agent tensioactif non ionique.

2. Composition pharmaceutique selon la revendication 1, **caractérisée en ce que** l'excipient est choisi dans le groupe constitué par les oligosaccharides, les disaccharides, les monosaccharides.

3. Composition pharmaceutique selon la revendication 2, **caractérisée en ce que** le monosaccharide est du glucose ou du fructose ou un mélange de ces monosaccharides.

4. Composition pharmaceutique selon la revendication 2, **caractérisée en ce que** le disaccharide est du saccharose, du maltose ou du lactose ou un mélange de ces disaccharides.

5. Composition pharmaceutique selon la revendication 1, **caractérisée en ce que** l'excipient est choisi parmi les sels.

6. Composition pharmaceutique selon la revendication 5, **caractérisé en ce que** les sels sont choisis parmi les citrates, les phosphates, les glutamates, les acétates.

7. Composition pharmaceutique selon l'une des revendications précédentes, **caractérisée en ce que** le sucre-alcool est du mannitol ou du sorbitol ou un mélange de ces sucres-alcools.

8. Composition pharmaceutique selon l'une des revendications 1 à 7, **caractérisée en ce que** l'acide aminé est la glycine, l'alanine, la valine, la leucine, la lysine, l'acide glutamique, l'acide aspartique, l'arginine, l'histidine ou un mélange de deux ou plusieurs des acides aminés cités.

9. Composition pharmaceutique selon la revendication 1, **caractérisée en ce que** l'agent tensioactif non ionique est du polysorbate, de l'octoxynol, des alcools polyoxyéthyléniques ou des esters polyoxyléthyléniques d'acides gras.

10. Composition pharmaceutique selon l'une des revendications précédentes, **caractérisée en ce qu'**elle présente un aspect colloïdal et comprend une huile métabolisable pharmaceutiquement acceptable telle que l'huile d'olive, de mais, de tournesol, de soja, de ricin, de squalène ou d'alphatocophérol.

11. Composition pharmaceutique selon l'une des revendications 1 à 10, **caractérisée en ce qu'**elle présente un aspect colloïdal et comprend une composition émulsionnée de lipides.

12. Composition pharmaceutique selon l'une des revendications précédentes, **caractérisée en ce que** la couleur est ajustée par addition d'un colorant autorisé ou d'un produit de réaction de Maillard allant du jaune au brun foncé.

13. Composition pharmaceutique selon l'une des revendications précédentes, **caractérisée en ce que** ladite composition présente une osmolarité comprise entre 250 et 650 milliosmoles après reconstitution sous forme de solution.

14. Composition pharmaceutique selon l'une des revendications précédentes, **caractérisée en ce qu'**elle est susceptible d'être obtenue à partir d'une solution de départ comprenant en pourcentage poids :
. excipient habituel 0,1 à 50% (PN)
. acide aminé ou sucre cristallisable 0,1 à 20% (PN)
. tensio-actif 10 à 200 ppm
. huile métabolisable ou composé hydrolipidique émulsionné et pigment (quantité suffisante pour avoir l'aspect du médicament).

15. Composition pharmaceutique selon l'une des revendications précédentes, **caractérisée en ce qu'**elle est sous la forme injectable.

16. Composition pharmaceutique selon l'une des revendications 1 à 14 **caractérisée en ce qu'**elle est sous la forme d'une application percutanée ou oculaire.

## Claims

1. Lyophilized pharmaceutical composition intended to imitate a medicinal product, based on proteins or peptides, said composition being free of said proteins or of said peptides and being intended to serve as a placebo, **characterized in that** it comprises a usual excipient chosen from amino acids which are in amorphous form in the solid state, one or more alcohol-sugars in crystalline form and/or one or more amino acids in crystalline form, and a nonionic surfactant.

2. Pharmaceutical composition according to Claim 1, **characterized in that** the excipient is chosen from the group consisting of oligosaccharides, disaccharides and monosaccharides.

3. Pharmaceutical composition according to Claim 2, **characterized in that** the monosaccharide is glucose or fructose or a mixture of these monosaccharides.

4. Pharmaceutical composition according to Claim 2, **characterized in that** the disaccharide is sucrose, maltose or lactose or a mixture of these disaccharides.

5. Pharmaceutical composition according to Claim 1, **characterized in that** the excipient is chosen from salts.

6. Pharmaceutical composition according to Claim 5, **characterized in that** the salts are chosen from citrates, phosphates, glutamates and acetates.

7. Pharmaceutical composition according one of the preceding claims, **characterized in that** the alcohol-sugar is mannitol or sorbitol or a mixture of these alcohol-sugars.

8. Pharmaceutical composition according to one of Claims 1 to 7, **characterized in that** the amino acid is glycine, alanine, valine, leucine, lysine, glutamic acid, aspartic acid, arginine, histidine or a mixture of two or more of the amino acids mentioned.

9. Pharmaceutical composition according to Claim 1, **characterized in that** the nonionic surfactant is polysorbate, octoxynol, polyoxyethylenic alcohols or polyoxyethylenic esters of fatty acids.

10. Pharmaceutical composition according to one of the preceding claims, **characterized in that** it has a colloidal aspect and comprises a pharmaceutically acceptable metabolizable oil such as olive oil, corn oil, sunflower oil, soybean oil, castor oil, squalene or α-tocopherol.

11. Pharmaceutical composition according to one of Claims 1 to 10, **characterized in that** it has a colloidal aspect and comprises an emulsified lipid composition.

12. Pharmaceutical composition according to one of the preceding claims, **characterized in that** the color is adjusted by adding an authorized colorant or a Maillard reaction product ranging from yellow to dark brown.

13. Pharmaceutical composition according to one of the preceding claims, **characterized in that** said composition has an osmolarity of between 250 and 650 milliosmoles after reconstitution in solution form.

14. Pharmaceutical composition according to one of the preceding claims, **characterized in that** it can be obtained from a starting solution comprising, as a percentage by weight:
• 0.1 to 50% (W/V) usual excipient
• 0.1 to 20% (W/V) crystallizable amino acid or sugar
• 10 to 200 ppm surfactant
• metabolizable oil or emulsified hydrolipidic compound and pigment (quantity sufficient to have the appearance of the medicinal product).

15. Pharmaceutical composition according to one of the preceding claims, **characterized in that** it is in injectable form.

16. Pharmaceutical composition according to one of Claims 1 to 14, **characterized in that** it is in the form of a percutaneous or ocular application.

## Patentansprüche

1. Lyophilisierte pharmazeutische Zusammensetzung, die dazu bestimmt ist, ein Medikament auf der Basis von Proteinen oder Peptiden nachzuahmen, wobei die Zusammensetzung frei von den Proteinen oder den Peptiden ist und dazu bestimmt ist, als Placebo zu dienen, **dadurch gekennzeichnet, dass** sie einen üblichen Hilfsstoff, der aus Aminosäuren ausgewählt ist, die im festen Zustand in amorpher Form vorliegen, einen oder mehrere Zuckeralkohole in kristalliner Form und/oder eine oder mehrere Aminosäuren in kristalliner Form und ein nichtionisches Tensid umfasst.

2. Pharmazeutische Zusammensetzung nach Anspruch 1, **dadurch gekennzeichnet, dass** der Hilfsstoff ausgewählt ist aus der Gruppe bestehend aus Oligosacchariden, Disacchariden, Monosacchariden.

3. Pharmazeutische Zusammensetzung nach Anspruch 2, **dadurch gekennzeichnet, dass** das Monosaccharid Glucose oder Fructose oder eine Mischung dieser Monosaccharide ist.

4. Pharmazeutische Zusammensetzung nach Anspruch 2, **dadurch gekennzeichnet, dass** das Disaccharid Saccharose, Maltose oder Lactose oder eine Mischung dieser Disaccharide ist.

5. Pharmazeutische Zusammensetzung nach Anspruch 1, **dadurch gekennzeichnet, dass** der Hilfsstoff aus Salzen ausgewählt ist.

6. Pharmazeutische Zusammensetzung nach Anspruch 5, **dadurch gekennzeichnet, dass** die Salze aus Citraten, Phosphaten, Glutamaten, Acetaten ausgewählt sind.

7. Pharmazeutische Zusammensetzung nach einem der vorangehenden Ansprüche, **dadurch gekennzeichnet, dass** der Zuckeralkohol Mannit oder Sorbit oder eine Mischung dieser Zuckeralkohole ist.

8. Pharmazeutische Zusammensetzung nach einem der Ansprüche 1 bis 7, **dadurch gekennzeichnet, dass** die Aminosäure Glycin, Alanin, Valin, Leucin, Lysin, Glutaminsäure, Asparaginsäure, Arginin, Histidin oder eine Mischung von zwei oder mehr der genannten Aminosäuren ist.

9. Pharmazeutische Zusammensetzung nach Anspruch 1, **dadurch gekennzeichnet, dass** es sich bei dem nichtionischen Tensid um Polysorbat, Octoxynol, Polyoxyethylenalkohole oder Polyoxyethylenester von Fettsäuren handelt.

10. Pharmazeutische Zusammensetzung nach einem der vorangehenden Ansprüche, **dadurch gekennzeichnet, dass** sie ein kolloidales Aussehen aufweist und ein pharmazeutisch annehmbares metabolisierbares Öl, wie Olivenöl, Maisöl, Sonnenblumenöl, Sojaöl, Rizinusöl, Squalenöl oder alpha-Tocopherolöl, umfasst.

11. Pharmazeutische Zusammensetzung nach einem der Ansprüche 1 bis 10, **dadurch gekennzeichnet, dass** sie ein kolloidales Aussehen aufweist und eine emulgierte Zusammensetzung von Lipiden umfasst.

12. Pharmazeutische Zusammensetzung nach einem der vorangehenden Ansprüche, **dadurch gekennzeichnet, dass** die Farbe durch Zugabe eines zugelassenen Färbemittels oder eines Produkts der Maillard-Reaktion eingestellt wird, wobei sie von gelb bis dunkelbraun reicht.

13. Pharmazeutische Zusammensetzung nach einem der vorangehenden Ansprüche, **dadurch gekennzeichnet, dass** die Zusammensetzung eine Osmolarität zwischen 250 und 650 Milliosmol einschließlich nach der Rekonstitution in Form einer Lösung aufweist.

14. Pharmazeutische Zusammensetzung nach einem der vorangehenden Ansprüche, **dadurch gekennzeichnet, dass** sie aus einer Ausgangslösung erhältlich ist, welche in Gewichtsprozent umfasst:
- üblichen Hilfsstoff, 0,1 -50 % (Gew./Vol.)
- Aminosäure oder kristallisierbaren Zucker, 0,1 bis 20 % (Gew.Nol.)
- Tensid, 10 bis 200 ppm
- metabolisierbares Öl oder emulgierte Hydrolipid-Zusammensetzung und Pigment (ausreichende Menge, um das Aussehen des Medikaments aufzuweisen).

15. Pharmazeutische Zusammensetzung nach einem der vorangehenden Ansprüche, **dadurch gekennzeichnet, dass** sie in injizierbarer Form vorliegt.

16. Pharmazeutische Zusammensetzung nach einem der Ansprüche 1 bis 14, **dadurch gekennzeichnet, dass** sie in Form einer perkutanen oder okularen Anwendung vorliegt.
